# EUROPEAN PATENT APPLICATION

(11) **EP 4 252 679 A1**
(43) Date of publication of application: **04.10.2023**
(21) Application number: 22871145.3
(22) Date of filing: 13.07.2022
(51) Int. Cl.: A61B 17/072, A61B 17/115

(54) **ACCESSORY FOR SURGICAL INSTRUMENT AND SURGICAL INSTRUMENT**

(30) Priority: 17.02.2022 CN 202210146475
(71) Applicant: NINGBO HITCM MEDICAL DEVICES CO., LTD., Ningbo, Zhejiang 315000 (CN)
(72) Inventor: LU, Shaolei, Ningbo, Zhejiang 315000 (CN); ZHANG, Zhixing, Ningbo, Zhejiang 315000 (CN)
(74) Representative: Viering, Jentschura & Partner mbB Patent- und Rechtsanwälte
(86) International application number: PCT/CN2022/105373
(87) International publication number: WO 2023/155370

(57) **Abstract**

The present disclosure describes an auxiliary unit for a surgical instrument, the surgical instrument includes an end effector, and the auxiliary unit includes a main member and a snap-fit member. The main member includes a base portion, a distal portion extending from the base portion, and a proximal portion extending from the base portion and configured to be engaged with the end effector. The snap-fit member is connected to the base portion and is detachably connected to the end effector. The present disclosure also discloses a surgical instrument including the auxiliary unit.

## Description

### TECHNICAL FIELD

The present disclosure relates to an auxiliary unit for a surgical instrument and a surgical instrument including such an auxiliary unit.

### BACKGROUND

Stapling instrument is a kind of surgical instrument, which can be used to replace traditional manual suture equipment. It has the advantages of rapid suture, simple operation as well as few side effects and surgical complications. A stapling instrument generally includes an end effector, a shaft assembly, and a handle portion. Additional auxiliary units may be provided on the end effector to facilitate various surgical procedures. For example, an eagle-beak-shaped dissection tool may be provided on the end effector to facilitate dissection of tissues in a confined space, followed by cutting and suturing of the dissected tissue using a stapling instrument. In existing products, the auxiliary unit is usually un-detachably secured to the end effector, so that a physician may need to replace different stapling instruments for operation during a surgery, causing inconvenience in use and high surgical costs.

### SUMMARY

In order to solve the above problems, the present disclosure proposes a detachable auxiliary unit and a surgical instrument including such an auxiliary unit.

According to one aspect of the present disclosure, there is provided an auxiliary unit for a surgical instrument, the surgical instrument includes an end effector, and the auxiliary unit includes a main member and a snap-fit member. The main member includes: a base portion, a distal portion extending from the base portion, and a proximal portion extending from the base portion and configured to be engaged with the end effector. The snap-fit member is connected to the base portion and is detachably connected to the end effector.

In some embodiments, each of opposite sides of the base portion may be provided with a hole and a first projection; the snap-fit member may include a connecting portion and a pair of lateral portions connected to both ends of the connecting portion and extending therefrom, respectively; each of the lateral portions includes a boss and a second projection. The boss may be configured to be rotatably fitted in the hole, and the second projection is configured to be snap-fitted with or separated from the first projection.

In some embodiments, the distal portion may be tapered along a direction from a proximal end to a distal end of the distal portion.

In some embodiments, an included angle between the distal portion and the base portion may be 15 degrees or 45 degrees.

In some embodiments, the proximal portion may include a cross-shaped column, a T-shaped column, a rectangular column, or a polygonal column.

In some embodiments, the first projection and the second projection may have a triangular prism shape.

In some embodiments, the second projection may be configured to slide over the first projection when applied with an external force so that the second projection and the first projection are snap-fitted with each other or separated from each other.

According to another aspect of the present disclosure, there is provided a surgical instrument including: an end effector including an anvil, the anvil including a channel penetrating therethrough along an axial direction thereof and a groove arranged at a distal end of the anvil; and the auxiliary unit according to the embodiments of the present disclosure. The proximal portion of the main member of the auxiliary unit is configured to be inserted into the channel, and the snap-fit member of the auxiliary unit is configured to be snap-fitted into the groove, so that the auxiliary unit is detachably connected to the anvil of the end effector.

In some embodiments, each of opposite sides of the base portion of the auxiliary unit is provided with a hole and a first projection; the snap-fit member of the auxiliary unit includes a connecting portion and a pair of lateral portions connected to both ends of the connecting portion and extending therefrom, respectively; each of the lateral portions includes a boss and a second projection. The boss is configured to be rotatably fitted in the hole, and the second projection is configured to slide over the first projection when applied with an external force so that the second projection and the first projection are snap-fitted with each other, and the connecting portion of the snap-fit member is configured to be snap-fitted in the groove.

In some embodiments, the proximal portion of the main member and the channel of the anvil are matched with each other in both a shape and a size, and are circumferentially fixed relative to each other.

### BRIEF DESCRIPTION OF DRAWINGS

Hereinafter, embodiments of the present disclosure will be further described by way of example with reference to the accompanying drawings which constitute a part of this specification. In the drawings:
Fig. 1 is a perspective view of a stapling instrument according to an exemplary embodiment;
Fig. 2 is a partially enlarged view of a circled portion of the stapling instrument of Fig. 1, illustrating an end effector and an auxiliary unit arranged on the end effector;
Fig. 3 is a perspective view of an auxiliary unit according to an exemplary embodiment in an assembled state;
Fig. 4 is a perspective view of an auxiliary unit according to an exemplary embodiment in a disassembled state;
Fig. 5 is a perspective view of another embodiment of an auxiliary unit according to an exemplary embodiment;
Fig. 6 is a perspective view of an end effector according to an exemplary embodiment, illustrating a channel arranged in an anvil of the end effector;
Fig. 7 is a perspective view of an end effector according to an exemplary embodiment, illustrating a groove arranged in an upper surface of an anvil; and
Fig. 8 is a perspective view of an auxiliary unit according to an exemplary embodiment, illustrating an engagement between a main member and a snap-fit member of the auxiliary unit in an installed state.

### DETAILED DESCRIPTION

Further features, advantages and technical effects of the present disclosure will be more clearly and thoroughly understood for those skilled in the art from the following detailed description of the embodiments of the present disclosure in conjunction with the accompanying drawings and specific implementations. In the following description, spatial and orientation terms such as "upper", "lower", "front", "rear", "top", "bottom", "vertical" and "horizontal" may be used to describe the embodiments of the present disclosure, but it should be understood that these terms are only for the convenience of explaining the embodiments illustrated in the drawings and are not intended to require a device to be actually constructed or operated in a specific orientation. In the following description, the use of terms such as "connected", "coupled", "fixed" and "attached" may refer to two elements or structures being directly connected without other elements or structures, or two elements or structures being indirectly connected through intervening elements or structures, unless explicitly stated otherwise herein. The use of ordinal numbers, such as "first" and "second", is only for distinguishing the cited elements from each other, and has no significance in their order or importance unless explicitly stated otherwise herein. As used herein, the terms "proximal/proximal end" and "distal/distal end" are defined relative to a clinician who manipulates a handle portion of a surgical instrument. The term "proximal/proximal end" refers to a part of an involved component or structure that is closer to the clinician, and the term "distal/distal end" refers to a part of the involved component or structure that is away from the clinician.

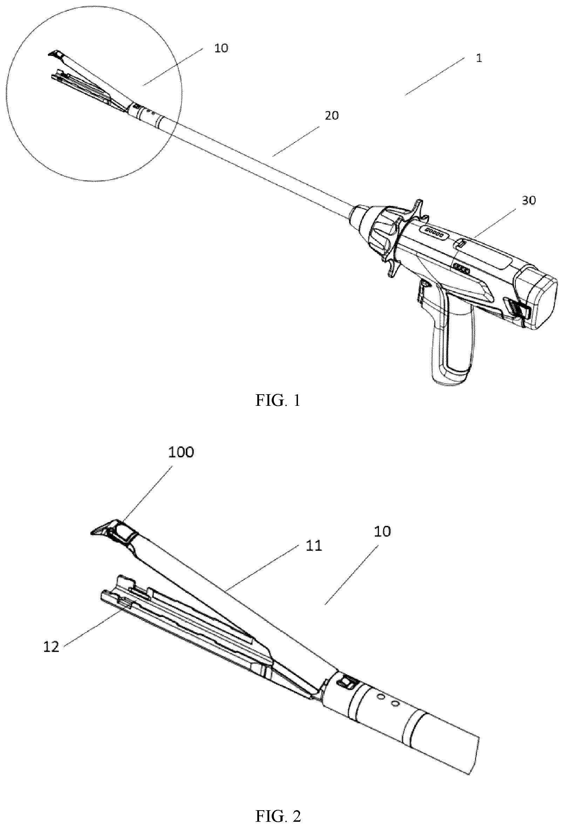

First of all, reference is made to Fig. 1, which is a perspective view of a stapling instrument 1 according to an exemplary embodiment. As illustrated in Fig. 1, the stapling instrument 1 includes an end effector 10 for performing a surgical procedure on a patient, a handle portion 30 held by a user, and a shaft assembly 20 connecting the end effector 10 and the handle portion 30. It should be noted that while the description herein is primarily directed to stapling instruments, those skilled in the art, in view of the disclosure and teachings of the present disclosure, may of course also apply the principles of the present disclosure to other types of surgical instruments.

Now turning to Fig. 2, which is a partially enlarged view of a circled portion of the stapling instrument 1 illustrated in Fig. 1, illustrating the end effector 10 and an auxiliary unit 100 arranged at a distal end of the end effector 10. As illustrated in Fig. 2, the end effector 10 includes a staple cartridge 12 for receiving staples and an anvil 11 pivotally connected to the staple cartridge 12 at a proximal end thereof. Through an action of a transmission mechanism in the shaft assembly 20, the anvil 11 is pivotable about the staple cartridge 12 between a closed position and an open position, so as to close and open the end effector 10. In alternative embodiments, the staple cartridge 12 may also be arranged to pivot relative to the anvil 11. In a surgery, it is sometimes necessary to dissect tissue in a confined space, and then the dissected tissue is cut and sutured using the stapling instrument. In this case, an auxiliary unit 100 may be provided on the end effector 10 to facilitate the dissection operation. The auxiliary unit 100 according to the present disclosure will be described in details below.

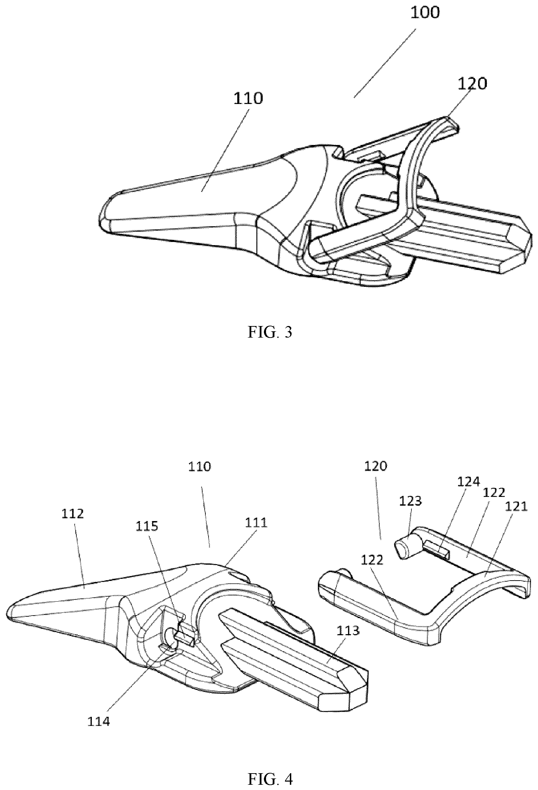

Referring to Figs. 3 and 4, which illustrate perspective views of the auxiliary unit 100 according to an exemplary embodiment in an assembled state and a disassembled state, respectively. As illustrated in the Figs. 3 and 4, the auxiliary unit 100 includes a main member 110 and a snap-fit member 120. The main member 110 includes a base portion 111, a distal portion 112 extending in a distal direction from the base portion 111, and a proximal portion 113 extending in a proximal direction from the base portion 111. In the illustrated embodiment, the distal portion 112 is configured to be tapered from a proximal end to a distal end thereof, and is in the shape of an eagle-beak. The eagle-beak-shaped distal portion 112 is narrower and more pointed compared with a distal end of an end effector 10 without an auxiliary unit, thus rendering it easier to access a narrow space or tubular incision, thereby reducing the difficulty of surgical manipulations. In alternative embodiments, other shapes of the distal portion 112 may of course be employed to satisfy operational requirements of different surgical procedures. When the stapling instrument is used to perform a surgery, for some surgical sites that are difficult to reach, such as blood vessels, the end effector can be placed at the positions where the blood vessels are located through a guidance of the distal portion 112, which is beneficial for the end effector to be placed and to clamp tissues, thereby improving the working efficiency and the convenience of the stapling instrument. The proximal portion 113 is configured to be engaged with the stapling instrument 1 and, in the illustrated embodiment, is in the form of a cross-shaped column, details of which will be described in more details below. As illustrated, the base portion 111 is further provided with a hole 114 and a first projection 115 in the form of triangular prism on each of two opposite sides thereof, expect a distal end and a proximal end thereof. It will be appreciated that, only the hole 114 and the first projection 115 at the visible side are labeled in Fig. 4, and similar hole and projection are likewise provided on the other side which is not visible. These one pair of holes 114 and one pair of first projections 115 are configured to be engaged with the snap-fit member 120, which will be described in details below.

As illustrated in the figure, the snap-fit member 120 is in the shape of a handle and includes a connecting portion 121 and one pair of lateral portions 122 which are connected with two ends of the connecting portion 121 and extending from the two ends of the connecting portion 121, respectively. Each of the lateral portions 122 includes an inwardly projected boss 123 at a distal end thereof and a triangular prism-shaped second boss 124 at an inner side thereof. It will be appreciated that, only the boss 123 and the second projection 124 at the visible side are labeled in Fig. 4, and similar boss and projection are likewise provided on the other side which is not visible. When the snap-fit member 120 is engaged with the main member 110, the boss 123 may be inserted into the hole 114 such that the snap-fit member 120 and the main member 110 are rotatably connected together. The snap-fitted connection between the first projection 115 and the second projection 124 may also be achieved by pressing the snap-fit member 120 downwards, as will be described in further details below.

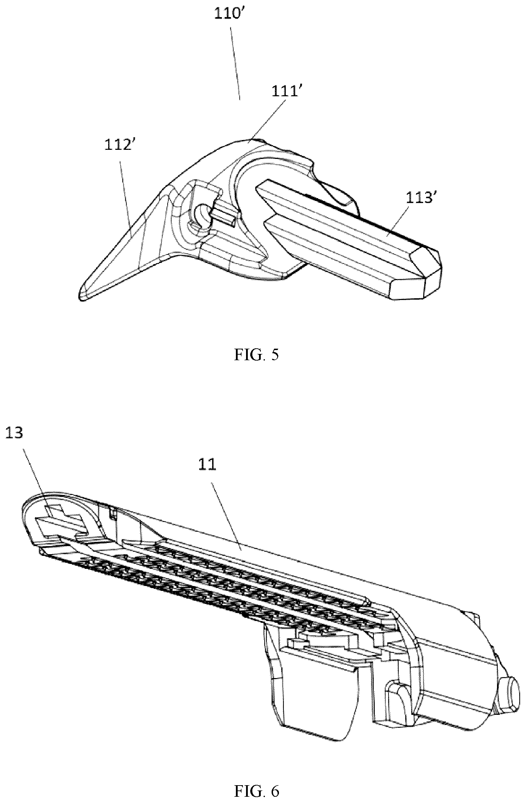

In the embodiment illustrated in Figs. 3 and 4, an included angle between the distal portion 112 and the base portion 111 is 15 degrees, which is defined as an angle between an upper surface of the distal portion 112 and the horizontal base portion 111 illustrated in the figures. Another embodiment of the auxiliary unit is illustrated in Fig. 5, respective features of which are substantially the same as the embodiments illustrated in Figs. 3 and 4, except that the included angle between the distal portion 112' and the base portion 111' of the auxiliary unit illustrated in Fig. 5 is 45 degrees, in order to accommodate different requirements of surgical manipulations. Since the auxiliary unit according to the present disclosure is detachable, auxiliary units of different included angles can be adopted as replacements as required during the surgery to facilitate the manipulations.

The engagement manner between the auxiliary unit and the end effector according to an exemplary embodiment will now be described in details with reference to Figs. 6-8.

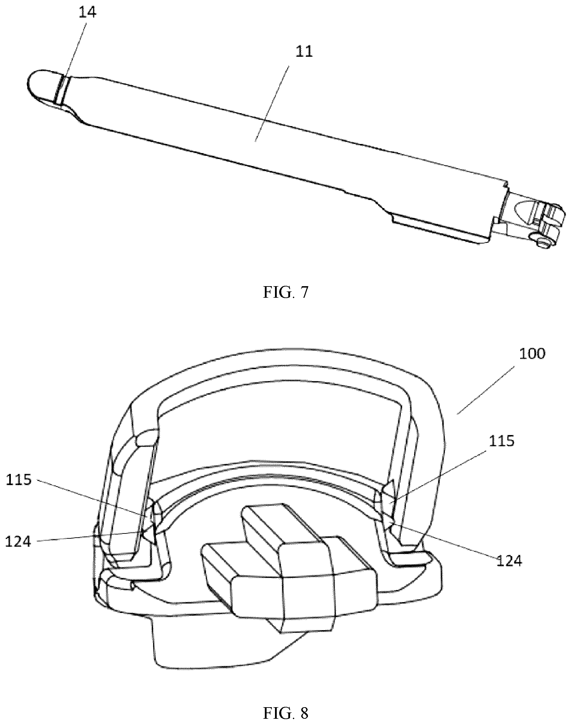

As illustrated in Fig. 6, the anvil 11 of the end effector 10 includes a channel 13 extending and penetrating through a main body thereof, the channel 13 has a shape corresponding to the shape of the proximal portion 113 in the form of cross-shaped column of the main member 110 of the auxiliary unit 100, so that the proximal portion 113 can be received in the channel 13. It should be understood that other shapes of the proximal portion 113 and the channel 13 may also be employed, as long as they can be engaged with each other. For example, the proximal portion 113 may also be presented as a T-shaped column, a rectangular column or a polygonal column in alternative embodiments, while the channel 13 may be shaped and dimensioned to match the proximal portion 113. As illustrated in Fig. 7, a surface of the anvil 11 facing away from the staple cartridge 12 has a distal end provided with a groove 14 which is adapted to receive the connecting portion 121 of the snap-fit member 120 of the auxiliary unit 100. It's conceivable for those skilled in the art that, similar structure as described above may also be provided at the distal end of other types of surgical instruments for installing the auxiliary unit according to the present disclosure.

Prior to installing the auxiliary unit 100 at the end effector 10, the snap-fit member 120 has been secured to the main member 110 by the engagement between the bosses 123 of the snap-fit member 120 and the holes 114 of the main member 110 as previously described, thereby completing the assembly of the auxiliary unit 100 itself. In installing the auxiliary unit 100, first of all, the operator inserts the proximal portion 113 into the channel 13 of the anvil 11, and then presses the snap-fit member 120 downwards so as to press the connecting portion 121 of the snap-fit member 120 into the groove 14 at the distal end of the anvil 11. As illustrated in Fig. 8, in the downward pressing process, the first projection 115 and the second projection 124 are abutted against each other, and the second projection 124 is pressed to slide over the first projection 115 and then reach a position below the first projection 115 under the action of an external force, so as to achieve the engagement between the first projection 115 and the second projection 124. In this way, the auxiliary unit 100 is snap-fitted with the anvil 11 by the snap-fit member 120. For detaching, a force is applied to take the connecting portion 121 out of the groove 14 so that the second projection 124 slides back to a position above the first projection 115 under the action of the external force, and the proximal portion 113 is withdrawn from the channel 13 of the anvil 11, thereby completing detaching the auxiliary unit 100. The installing and detaching processes are very simple and convenient, and are beneficial for users to decide whether to use an auxiliary unit or to replace different types of auxiliary units according to different requirements during the surgical procedures.

In summary, an auxiliary unit for a surgical instrument and a surgical instrument including such an auxiliary unit are provided. The auxiliary unit according to the present disclosure is detachably installed at a surgical instrument, whereby it is possible to decide whether to use an auxiliary unit or to use different types of auxiliary units to facilitate a surgical procedure as desired. The auxiliary unit according to the present disclosure also has the advantages of simple structure, easy disassembly and assembly, and the like.

Although the present disclosure has been described with reference to the above embodiments, those skilled in the art will understand that various changes can be made without departing from the concept and scope of the present disclosure as defined in the appended claims. Although the specification contains many specific implementation details, these should not be construed as limitations on the scope of the present disclosure, but as descriptions of features specific to particular embodiments. The scope of the present disclosure is defined by the appended claims and their equivalents, and is not limited to the previously described embodiments.

## Claims

1. An auxiliary unit for a surgical instrument, the surgical instrument comprising an end effector, wherein the auxiliary unit comprising:
a main member, comprising:
a base portion,
a distal portion extending from the base portion, and
a proximal portion extending from the base portion, the proximal portion being configured to be engaged with the end effector; and
a snap-fit member connected to the base portion and detachably connected to the end effector.

2. The auxiliary unit according to claim 1, wherein
each of opposite sides of the base portion is provided with a hole and a first projection,
the snap-fit member comprises a connecting portion, and a pair of lateral portions connected to both ends of the connecting portion and extending therefrom, respectively,
each of the lateral portions comprises a boss and a second projection, wherein
the boss is configured to be rotatably fitted in the hole, and the second projection is configured to be snap-fitted with or separated from the first projection.

3. The auxiliary unit according to claim 1, wherein the distal portion is tapered along a direction from a proximal end to a distal end of the distal portion.

4. The auxiliary unit according to claim 1, wherein an included angle between the distal portion and the base portion is 15 degrees or 45 degrees.

5. The auxiliary unit according to claim 1, wherein the proximal portion comprises a cross-shaped column, a T-shaped column, a rectangular column or a polygonal column.

6. The auxiliary unit according to claim 1, wherein both the first projection and the second projection have a triangular prism shape.

7. The auxiliary unit according to claim 2, wherein the second projection is configured to slide over the first projection when applied with an external force, so that the second projection and the first projection are snap-fitted with each other or separated from each other.

8. A surgical instrument, comprising:
an end effector comprising an anvil, the anvil comprising a channel penetrating therethrough along an axial direction thereof and a groove arranged at a distal end of the anvil; and
the auxiliary unit according to any of claims 1 to 7, wherein the proximal portion of the main member of the auxiliary unit is configured to be inserted into the channel, and the snap-fit member of the auxiliary unit is configured to be snap-fitted into the groove, so that the auxiliary unit is detachably connected to the anvil of the end effector.

9. The surgical instrument according to claim 8, wherein
each of opposite sides of the base portion of the auxiliary unit is provided with a hole and a first projection,
the snap-fit member of the auxiliary unit comprises a connecting portion and a pair of lateral portions connected to both ends of the connecting portion and extending therefrom, respectively,
each of the lateral portions comprises a boss and a second projection, wherein
the boss is configured to be rotatably fitted in the hole, and the second projection is configured to slide over the first projection when applied with an external force, so that the second projection and the first projection are snap-fitted with each other, and
the connecting portion of the snap-fit member is configured to be snap-fitted in the groove.

10. The surgical instrument according to claim 8, wherein the proximal portion of the main member and the channel of the anvil are matched with each other in both a shape and a size, and are circumferentially fixed relative to each other.
